# EUROPÄISCHE PATENTANMELDUNG

(11) **EP 2 394 675 A2**
(43) Veröffentlichungstag der Anmeldung: **14.12.2011**
(21) Anmeldenummer: 11169459.2
(22) Anmeldetag: 10.06.2011
(51) Int. Cl.: A61L 27/54, A61C 7/00, A61C 8/00

(54) **Zahnmedizinische Apparatur**

(30) Priorität: 10.06.2010 EP 10165541
(71) Anmelder: Novak, Velimir-Josef, 6033 Buchrain (CH)
(72) Erfinder: Novak, Velimir-Josef, 6033 Buchrain (CH)
(74) Vertreter: OK pat AG

(57) **Zusammenfassung**

Zahnmedizinische Apparatur zur Montage im Mund (100) dadurch gekennzeichnet, dass sie ein antibakteriell wirkendes Depot (10) umfasst, das kontinuierlich abgebbare Silber-Ionen beinhaltet.

## Beschreibung

### ERFINDUNGSGEBIET

Gegenstand der Erfindung ist eine zahnmedizinische Apparatur zur Montage im Mund eines Menschen oder Tieres, die Infektionen durch Bakterien, bakterielle Beläge und dergleichen effektiv verhindern kann.

Es wird die Priorität der europäischen Patentanmeldung EP 10 165 541.3 beansprucht, die am 10. Juni 2010 mit dem Titel "Zahnmedizinisches Implantsystem" beim Europäischen Patentamt hinterlegt worden ist.

### STAND DER TECHNIK

Der menschliche und tierische Köper ist einer Vielzahl verschiedener Bakterien ausgesetzt. Dabei ist der Mund einer der Haupteintrittsorte für Bakterien.

Schädliche Bakterien wurden bislang mit Antibiotika mehr oder weniger erfolgreich therapiert, wobei stets Nebenwirkungen, Unverträglichkeiten und Resistenzen aufgetreten sind. Bei vielen Antibiotika sind gastrointestinale Störungen wie Übelkeit, Erbrechen, Durchfall sehr häufig. Auch allergische Reaktionen sind verbreitet und verbieten in vielen Fällen sogar eine antibiotische Behandlung.

Im menschlichen und tierischen Mundraum sind zahlreiche Bakterienstämme anzutreffen, deren Stoffwechsel- und Zerfallsprodukte als bakterielle Plaque die Zähne langsam überziehen. Durch Plaque werden körpereigene Abwehrreaktionen gegen die im Mundraum siedelnden Bakterien ausgelöst, wobei Enzyme freigeschaltet werden, die versuchen, die Bakterien zu zerstören, und ein Vordringen der Mikroorganismen in tiefere Gewebeschichten zu verhindern, jedoch stattdessen zu einer Zerstörung des Eigengewebes führen können. So kann es selbst bei täglich durchgeführter sorgfältiger Mundhygiene durch die Nebeneffekte der körpereigenen Immunreaktion zu Entzündungen und /oder Infektionen im Mundraum kommen.

Die Gefahr einer Infektion ist besonders hoch, wenn Fremdkörper wie z. B. eine zahnmedizinische Apparatur im Mund angebracht werden. Dies ist der Fall bei Zahnimplantaten und Zahnspangen.

Bei Zahnimplantaten ist die Einheilungsphase besonders kritisch, weil ein Fremdkörper in den Kieferknochen eingebracht wird und dadurch das Zahnfleisch und die umliegenden Bereiche stark beschädigt werden. Deswegen ist das Risiko einer Infektion der Wunde und/oder Schwellungen des Zahnfleisches erhöht. Da die Einheilphase bis zu 6 Monate dauern kann, ist eine dauernde Bekämpfung der Infektionen durch Antibiotika oder andere Arzneimittel nicht bevorzugt, weil nach einiger Zeit eine Resistenz gegen die Antibiotika oder andere Arzneimittel auftreten kann. Auch die moderne Ernährung führt zu einer zusätzlichen Vermehrung von Bakterien in Mundbereich.

Im Falle von Zahnspangen ist wegen der sehr langen Dauer der Behandlung die Bekämpfung der Infektionen durch Antibiotika ausgeschlossen.

Die nachteiligen Folgen regelmässiger Antibiotikabehandlungen sind bekannt; insbesondere die zunehmende Resistenz zahlreicher Bakterienstämme gegenüber häufig verwendeten Antibiotika ist hierbei sehr problematisch. Trotzdem können während der Einsatzzeit verschiedene Infektionen auftreten, die, wenn nicht sofort behandelt, zu Komplikationen führen können.

Eine antibakterielle Wirkung wird neben den bereits genannten Antibiotika auch z.B. durch Silber-Ionen erreicht. Diese antibakterielle Wirkung des Silbers erklärt sich daraus, dass in feuchtem Umfeld Silber-Ionen freigesetzt werden, die die Zellwände von Bakterien in der näheren Umgebung angreifen und zerstören. Damit hat Silber eine antibakterielle Wirkung, die praktisch nebenwirkungsfrei zur Anwendung kommen kann. Die therapeutische Verwendungsform von Silber ist in der Medizin als kolloidales Silber bekannt. Bei kolloidalem Silber handelt es sich entweder um flüssige Dispersionen elementaren Silbers oder schwerlöslicher Silberverbindungen. Diese Silberkolloide bzw. Silbersole sind von Lösungen löslicher Silbersalze zu unterscheiden. Die Teilchengrössen liegen zwischen 1 und 100 nm und sind weder mit dem Auge noch mit einem Lichtmikroskop erkennbar. In den einzelnen Teilchen sind etwa 1.000 bis 1 Milliarde Silberatome oder Moleküle der entsprechenden Silberverbindung enthalten.

Verschiedene antibakterielle Zahnpasten (siehe z. B. US 6 123 925), Zahnbürsten (siehe z. B. DE 19508539), Zungenreiniger (siehe z. B. DE 29803277 U1) sind bekannt, die Silber-Ionen in der einen oder anderen Form beinhalten, um eine gewisse antibakterielle Wirkung zu gewährleisten. Alle diese bekannte antibakteriellen Anwendungen von Silber-Ionen basieren auf einer hohen Silberkonzentration, die sehr schnell ausgelöst wird, um ihre Wirkung erfüllen zu können. Leider verursachen solche hohen Konzentrationen von sich schnell lösendem kolloidalem Silber unerwünschte Nebeneffekte wie schlechten Geschmack oder allergische Reaktionen. Ferner ist die Wirkung solcher kurzfristigen "Behandlungen" sehr fraglich.

### ZU LÖSENDE TECHNISCHE AUFGABE

Die technische Aufgabe der Erfindung ist daher ein zahnmedizinische Vorrichtung bereitzustellen, die Infektionen durch Bakterien dauerhaft verhindern kann und dabei keine unerwünschte Nebeneffekte verursachen.

### OFFENBARUNG DER ERFINDUNG

Die oben genannte Aufgabe der Erfindung ist durch eine zahnmedizinische Apparatur zur Montage im Mund gelöst, die dadurch gekennzeichnet ist, dass sie ein antibakteriell wirkendes Depot umfasst, das kontinuierlich abgebbare Silber-Ionen beinhaltet.

Weitere vorteilhafte Ausführungsformen der Erfindung werden in den Unteransprüchen definiert.

Die Erfindung basiert auf der Erkenntnis, dass kolloidales Silber die Fähigkeit hat silberempfindliche Bakterien, Viren und Pilze nach ausreichend langer Einwirkzeit sowie bei ausreichend hoher Silberkonzentration zu inaktivieren. Die Erfindung setzt dabei jedoch auf Konzentrationen der Ionen, die deutlich unter den Konzentrationen der eingangs erwähnten oral zu verwendenden Zahnpasten, Zahnbürsten und Zungenreiniger liegen.

### VORTEILHAFTE WIRKUNGEN

Der wichtigste Vorteil der vorliegenden Erfindung ist, dass die antibakterielle Wirkung dauerhaft ist und während des ganzen Heilung-/ Behandlungsprozesses aufrechterhaltbar ist. Das heisst, dass die Silber-Ionen von dem Depot in einer niedrigen Konzentration kontinuierlich abgegeben werden, um einen konstanten Schutz gegen Bakterien und dadurch gegen Infektionen bereitzustellen.

Ein weiterer Vorteil ist, dass gemäss einer weiteren Ausführungsform der Erfindung, das Depot, das die kontinuierlich abgebbaren Silber-Ionen beinhaltet, unabhängig von einem zahnmedizinischen Implantatsystem austauschbar / erneuerbar ist. Ein solcher Austausch / Erneuerung ist notwendig, wenn die Einsatzdauer des zahnmedizinischen Implantatsystems länger ist als die Kapazität des Depots ermöglicht, d.h. die Fähigkeit zum Abgeben von Silber-Ionen des Depots erschöpft ist.

Im Falle eines Depots in Form einer Beschichtung ist eine Erneuerung dieser Beschichtung erforderlich. Im Falle eines Depots in Form eines Implantatsystems ist ein schneller und unkomplizierter Austausch des Implantatsystems möglich, um die antibakterielle Wirkung aufrechtzuerhalten.

### KURZE BESCHREIBUNG DER ZEICHNUNGEN

Weitere Einzelheiten und Vorteile der Erfindung werden im Folgenden anhand von Ausführungsbeispielen und mit Bezug auf die Zeichnung beschrieben.
- Fig. 1A: zeigt eine vereinfachte Seitenansicht der ersten Ausführungsform der vorliegenden Erfindung, wobei das Depot in Form eines Implantatsystems angebracht ist;
- Fig. 1B: zeigt eine vereinfachte Draufsicht der ersten Ausführungsform der vorliegenden Erfindung, wobei das Depot in Form eines Implantatsystems angebracht ist;
- Fig. 1C: zeigt eine vereinfachte Schnittansicht der ersten Ausführungsform der vorliegenden Erfindung, wobei das Depot in Form eines Implantatsystems angebracht ist;
- Fig. 2A: zeigt eine vereinfachte Seitenansicht einer weiteren Ausführungsform der vorliegenden Erfindung, bei dem die zahnmedizinische Apparatur ein zweites antibakteriell wirkendes Depot umfasst, wobei sich zwischen dem Depot und dem zweiten Depot nach der Montage in Mund eine niedrige elektrische Spannungsdifferenz ergibt und dadurch eine besonders effektive antibakterielle Wirkung gewährleistet wird;
- Fig. 2B: zeigt eine vereinfachte Draufsicht der weiteren Ausführungsform der vorliegenden Erfindung aus der Fig. 2A;
- Fig. 3A: zeigt eine vereinfachte Seitenansicht einer weiteren Ausführungsform der vorliegenden Erfindung, wobei es sich um ein Zahnimplantat handelt und wobei das Depot an dem Zahnimplantat befestigbar ist;
- Fig. 3B: zeigt eine vereinfachte Draufsicht der weiteren Ausführungsform der vorliegenden Erfindung aus der Fig. 3A;
- Fig. 4A: zeigt eine Perspektivenansicht einer weiteren Ausführungsform der vorliegenden Erfindung, wobei es sich um eine Zahnspange handelt;
- Fig. 4B: zeigt eine vereinfachte Frontansicht der weiteren Ausführungsform der vorliegenden Erfindung aus der Fig. 4A;
- Fig. 4C: zeigt eine Perspektivenansicht der weiteren Ausführungsform der vorliegenden Erfindung, wobei es sich um eine Zahnspange handelt und bei dem die zahnmedizinische Apparatur ein zweites antibakteriell wirkendes Depot umfasst, wobei sich zwischen dem Depot und dem zweiten Depot nach der Montage in Mund eine niedrige elektrische Spannungsdifferenz ergibt und dadurch eine besonders effektive antibakterielle Wirkung gewährleistet wird;
- Fig. 4D: zeigt eine vereinfachte Frontansicht der weiteren Ausführungsform der vorliegenden Erfindung aus der Fig. 4C.

### DETAILLIERTE BESCHREIBUNG DER BEVORZUGTEN AUSFÜHRUNGSFORMEN

In dieser Patentanmeldung werden bestimmte Begriffe benutzt, deren Interpretation nicht auf den spezifisch gewählten Begriff beschränkt werden soll. Diese Begriffe beziehen sich vielmehr auf das allgemeinen Konzept dahinter.

Der Begriff "zahnmedizinische Apparatur" bezieht sich auf jede Art im Mund oder Kieferbereich angebrachter fremder Körper wie Zahnspangen, Zahnimplantate, Brücken, Inserts, Inlays oder sogar Füllungen.

"Mund" gemäss der Erfindung bedeutet den Mund eines Menschen oder Tieres. Die zahnmedizinische Apparatur der vorliegenden Erfindung kann nicht nur bei Menschen, sondern auch bei vielen Tierarten Anwendung finden, die häufig unter Zahn- und Mundproblemen leiden, wie z.B. Pferden, Hunden oder Katzen.

Der Begriff "Depot" beinhaltet verschiedene Arten von Reservoirs, Kapseln, Behälter aber auch Beschichtungen, die eine Masse von Materie speichern oder tragen können. Besonders als Depot bevorzugt sind jedoch Reservoirs, Kapseln oder Behälter.

Mit dem Begriff "kontinuierlich abgebbar" ist gemeint, dass ein Stoff, Materie über längere Zeit, vorzugsweise in kleinen Dosen, abgeben kann. Diese "längere Zeit" kann, entsprechend des Einsatzbereichs, eine Zeitspanne von einigen Tagen bis zu einigen Monaten dauern. "kontinuierlich abgebbar" bedeutet insbesondere, dass mindestens über einen Zeitraum von mehr als 24 Stunden eine mehr oder weniger gleichmässige Menge an Ionen abgebbar ist. Diese Forderung der gleichmässig abzugebenden Menge an Ionen hängt jedoch von den Umgebungsbedingungen ab. Dabei ist zu berücksichtigen, dass es im Mundraum, z.B. aufgrund des unterschiedlichen Speichelflusses, sich ändernde Bedingungen geben kann. Die Forderung in Bezug auf die gleichmässig abzugebenden Menge bezieht sich auf einen Testaufbau im Labor mit gleichbleibenden Umgebungsbedingungen. D.h. die Mittel der Erfindung müssen bei allen Ausführungsformen so ausgelegt sein, dass sie im Labor bei gleichbleibenden Umgebungsbedingungen über einen Zeitraum von mehr als 24 Stunden eine mehr oder weniger gleichmässige Menge an Ionen abgeben.

Der Kern der vorliegenden Erfindung ist das sogenannte Depot 10, das kontinuierlich dosierbare Silber-Ionen, auch als kolloidales Silber bekannt, beinhaltet. Kolloidales Silber ist eine therapeutische Verwendungsform von Silber, die in verschiedenen Formen zum Einsatz kommt. Bei kolloidalem Silber handelt es sich entweder um eine flüssige Dispersionen elementaren Silbers oder schwerlöslicher Silberverbindungen. Diese Silberkolloide, auch Silbersole genannt, sind von Lösungen löslicher Silbersalze zu unterscheiden. Die Teilchengrössen liegen zwischen 1 und 100 nm.

Präzis dosierte kleine Mengen an Silber-Ionen haben eine schädigende Wirkung auf lebende Zellen und können den Stoffwechsel mancher Bakterien stören. Die durch Silber-Ionen induzierte antimikrobielle Wirkung tritt am schnellsten ein, wenn die wirksame Oberfläche, auch Interaktions- oder Wirkfläche genannt, sehr gross ist, was bei Silberkolloiden der Fall ist.

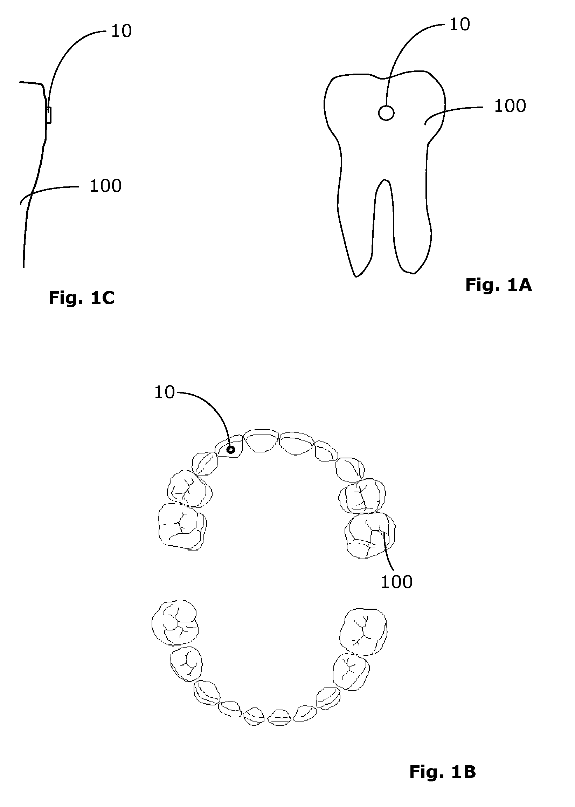

Fig. 1A zeigt eine vereinfachte Seitenansicht der ersten Ausführungsform der zahnmedizinischen Apparatur, wobei das Depot 10 in Form einer Kapsel angebracht ist. Das Depot 10 in Form eine Kapsel besteht aus einer Kapselhülle und einer Füllung. Die Hülle besteht aus einem oder mehreren der folgenden unterschiedlich lösbaren Materialien, wie Gelatine, Cellulose oder Carrageen, kann aber selbst auch aus Silber gefertigt sein. Die Füllung ist entweder reines kolloidales Silber oder eine Matrix aus Metall, Kunststoff (z.B. ein Polymerkunststoff) und/oder Keramik, die mit kolloidalem Silber benetzt, versetzt, beschichtet oder befüllt ist.

Wie in der Figur 1A zu erkennen ist, werden die Depots 10 so dimensioniert, dass sie in den bestimmten Einsatzbereich passen. In dem aufgezeigten Fall ist das Depot 10 an die Grösse eines Zahnes 100 angepasst.

In der Figur 1B ist eine vereinfachte Draufsicht der ersten Ausführungsform zu sehen, wobei das Depot 10 in Form einer Kapsel innenliegend hinter einer Zahnreihe angebracht ist. In der Figur 1C ist eine entsprechende seitliche Schnittansicht gezeigt.

Vorzugsweise hat das Depot 10 bei allen Ausführungsformen eine optisch ansprechende und/oder anatomisch günstige Form. Besonders bevorzugt sind leicht runde, bombierte oder gewölbte oder taschenartige Formen.

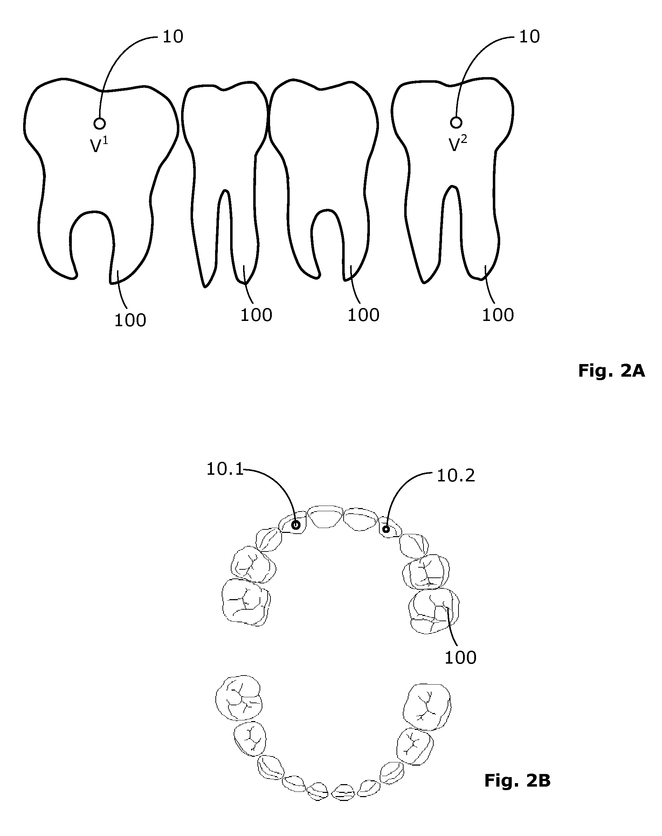

Figur 2A zeigt eine vereinfachte Seitenansicht einer weiteren Ausführungsform der zahnmedizinischen Apparatur. Die zahnmedizinische Apparatur umfasst hier ein erstes antibakteriell wirkendes Depot 10.1 und ein zweites antibakteriell wirkendes Depot 10.2, wobei sich zwischen dem ersten Depot 10.1 und dem zweiten Depot 10.2 nach der Montage in Mund eine niedrige elektrische Spannungsdifferenz ergibt und dadurch eine besonders effektive antibakterielle Wirkung gewährleistet wird. Die elektrische Spannung ergibt durch einen Potenzialunterschied zwischen dem Potenzial V¹ des ersten Depots 10.1 und dem Potenzial V² des zweiten Depots 10.2. Das Depot 10.1 kann z.B. aus einem edlen Metall (hier Silber oder eine Silberlegierung) und das Depot 10.2 aus einem weniger edlen Metall gefertigt sein. Wegen der elektrischen Leitfähigkeit des Speichels und den darin enthaltenen Elektrolyten, ergibt sich zwischen dem ersten Depot 10.1 und dem zweiten Depot 10.2 im Mund eine niedrige Spannungsdifferenz. Diese niedrige Spannungsdifferenz löst kontinuierlich weitere Silber-Ionen aus dem/den Depots 10.1, 10.2 und sichert dadurch eine besonders effektive antibakterielle Wirkung der zahnmedizinischen Apparatur.

Eine Draufsicht derselben zahnmedizinischen Apparatur mit einem weiteren Depot 10.2 ist in der Fig. 2B zu sehen, wobei die aufgezeigte Platzierung der Depots 10.1, 10.2 nur als Illustration gilt und nicht einschränkend ausgelegt werden soll.

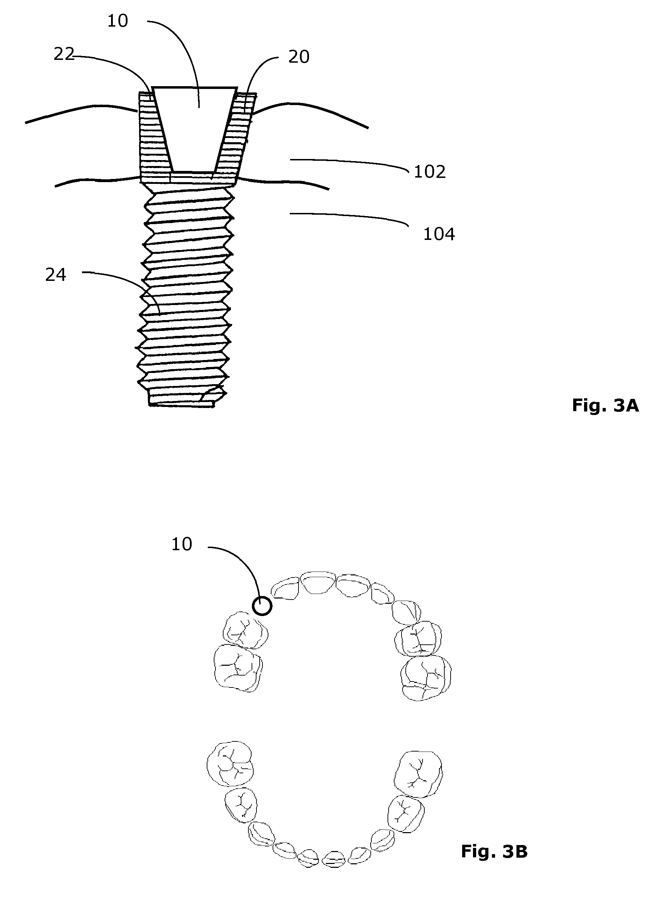

Figur 3A zeigt eine vereinfachte Seitenansicht einer weiteren Ausführungsform der zahnmedizinischen Apparatur gemäss der vorliegenden Erfindung, wobei es sich bei der Apparatur um ein Zahnimplantat 20 handelt und wobei das Depot 10 an dem Zahnimplantat 20 befestigbar (im Sinnen von integier- oder einbaubar) ist oder ein integraler Bestandteil des Zahnimplantats 20 ist. Wie auf der Figur 3A zu erkennen ist, wird das Zahnimplantat 20 durch das Zahnfleisch 102 in den Kieferknochen 104 eingebracht (meistens eingeschraubt). In der in Figur 3A illustrierten Ausführungsform wird das Depot 10 an dem Zahnimplantat 20 befestigt, um Infektionen während der Einheilphase des Zahnimplantats 20 zu verhindern. Das Depot 10 ist so konfiguriert, dass es in ein inneres Gewinde 22 des Implantatkörpers 24 hineinpasst. Dadurch wird sichergestellt, dass das Depot 10 im Mundraum 100 nicht stört und dass die antibakterielle Wirkung genau dort fokussiert ist, wo sie am meisten benötigt ist, nämlich rundum die Stelle, wo zuvor ein Fremdkörper (hier das Zahnimplantat 20) angebracht worden ist.

Zusätzlich kann das Zahnimplantat 20 bei allen Ausführungsformen mit einer Beschichtung aus kolloidalem Silber versehen werden, die kontinuierlich abgebbare Silber-Ionen beinhaltet.

Fig. 3B zeigt eine vereinfachte Draufsicht der weiteren Ausführungsform der vorliegenden Erfindung aus der Fig. 3A, wobei die Kontur des in dem Implantatkörper 24 befestigten Depots 10 gut erkennbar ist.

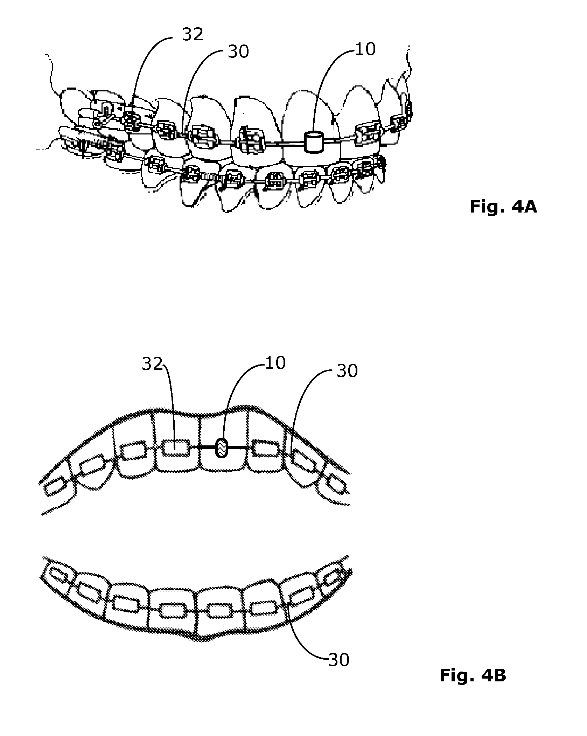

Figur 4A zeigt eine Perspektivansicht einer weiteren Ausführungsform der zahnmedizinischen Apparatur gemäss der vorliegenden Erfindung, wobei es sich um eine Zahnspange 30 handelt. Es kann sich um verschiedene Arten von Zahnspangen 30 handeln, wie eine Multiband- bzw. Multibracketapparatur, ein Herbstscharnier oder eine Delaire-Maske. Die verwendeten Materialien der Multibracketapparaturen (Zahnklammer) reichen von Edelstahl über Titan, Kunststoff bis hin zum durchsichtigen Keramikbrackets.

Ein wesentliches Problem der Zahnspange 30 ist die Tatsache, dass die Spange 30 über viele Monate oder Jahre ununterbrochen getragen werden muss und auch nicht in Ausnahmefällen einfach abgenommen werden kann. Dies führt sehr oft zu Infektionen. Dieses Problem wird zu Behandlungsbeginn vielfach als zu gering eingeschätzt bzw. kann aufgrund des langen Zeitraums oftmals nicht objektiv abgeschätzt werden. Das Problem wird mittels der vorliegenden Erfindung beseitigt, indem die kontinuierlich abgegebenen Silber-Ionen die Bakterien ständig bekämpfen. Wie in der Figur 4A zu sehen ist, ist das Depot 10 an der Zahnspange 30 in Form einer Kapsel befestigt. Das Depot 10 in Form einer Kapsel besteht vorzugsweise aus einer Kapselhülle und einer Füllung. Die Hülle kann aus einem oder mehreren unterschiedlich lösbaren Materialien, wie Gelatine, Cellulose oder Carrageen, kann aber selbst auch aus Silber gefertigt sein. Die Füllung ist entweder reines kolloidales Silber oder eine Matrix aus Metall, Kunststoff (z.B. ein Polymerkunststoff) und/oder Keramik, die mit kolloidalem Silber benetzt, versetzt, beschichtet oder befüllt ist.

Das Depot 10 in Form einer Kapsel kann nachträglich an der Aussenseite aber auch an der Innenseite der Zahnspange 30, besonders bei sogenannten lingualen Zahnspangen, montiert werden, gemäss dem jeweiligen spezifischen Einsatz. Das Depot 10 kann auch bereits bei der Herstellung in die Zahnspange 30 integriert werden. Die Grösse und die Form des Depots 10 werden so konfiguriert, dass das Depot 10 im Mundraum nicht stört.

Bei Multibracketapparaturen kann das Depot 10 in einer oder mehreren der Brackets 32 integriert sein. Dadurch benötigt das Depot 10 keines zusätzlichen Platz im Mundraum.

In der Figur 4B ist eine Frontansicht der weiteren Ausführungsform der vorliegenden Erfindung aus der Fig. 4A zu sehen, wobei das Depot 10 in eines oder in mehrere der Brackets 32 integriert ist.

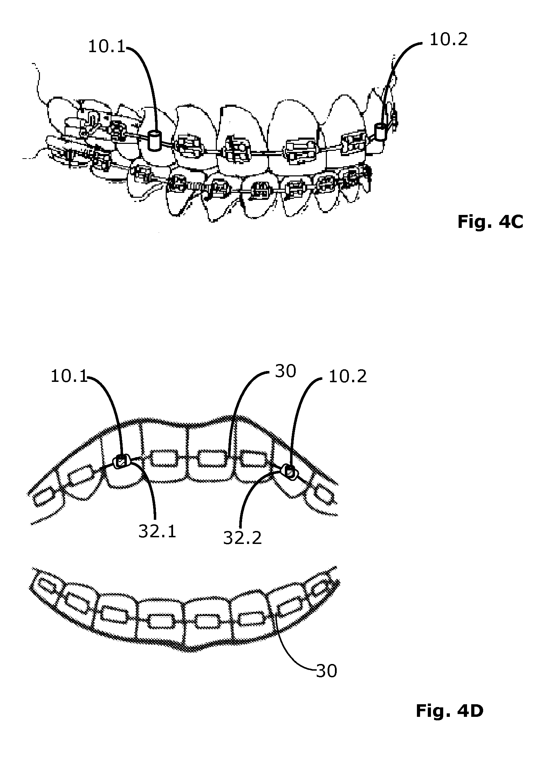

Fig. 4C zeigt eine Perspektivenansicht der weiteren Ausführungsform der zahnmedizinischen Apparatur gemäss der vorliegenden Erfindung, wobei es sich um eine Zahnspange 30 handelt. Die zahnmedizinische Apparatur umfasst zusätzlich zu dem ersten antibakteriell wirkenden Depot 10.1 ein zweites antibakteriell wirkendes Depot 10.2, wobei sich zwischen dem Depot 10.1 und dem zweiten Depot 10.2 nach der Montage in Mund 100 eine niedrige elektrische Spannungsdifferenz ergibt, wie bereits beschrieben, und dadurch eine besonders effektive antibakterielle Wirkung gewährleistet wird. Wichtig ist in einem solchen Fall, dass es keine elektrisch leitende Verbindung durch das Metall der Zahnspange 30 gibt, weil sich in diesem fall keine elektrische Spannungsdifferenz aufbauen kann. Der Bereich in dem sich das erste antibakteriell wirkende Depot 10.1 befindet, muss elektrisch gegenüber dem Bereich isoliert sein, in dem sich das zweite antibakteriell wirkende Depot 10.2 befindet.

Figur 4D zeigt eine Frontansicht der weiteren Ausführungsform der vorliegenden Erfindung aus der Fig. 4C, wobei beide Depots 10.1, 10.2 in entsprechenden Brackets 32.1, 32.2 integriert sind.

Zusätzlich kann auch die Zahnspange 30 oder nur die einzelnen Brackets 32 mit einer Beschichtung aus kolloidalem Silber versehen werden, die kontinuierlich abgebbare Silber-Ionen beinhaltet.

Das gemeinsame erfinderische Konzept der Erfindung, die kontinuierlich abgebbaren Silber-Ionen, können in verschiedenen Formen zum Einsatz kommen:
- als flüssige Dispersionen elementaren Silbers oder schwerlöslicher Silberverbindungen und/oder
- als Silbersalze, wie z. B. Silberfluorid (AgF), Silberchlorid (AgCl), Silberbromid (AgBr) und Silberiodid (AgI), Silbernitrat AgNO₃, Silberfulminat (Knallsilber, AgCNO), Silberazid AgN₃ und Silbersulfid Ag₂S und/oder
- einbettet, benetzt, versetzt, beschichtet oder befüllt in einer Matrix aus Metall, Kunststoff und/oder Keramik.

Vorzugsweise ist bei allen Ausführungsformen, die oben präsentiert worden sind, das Depot 10 unabhängig von der zahnmedizinischen Apparatur austauschbar/ erneuerbar. Im Falle des Depots 10 in Form einer Kapsel, wird diese Kapsel nach Erreichen der Kapazitätsgrenze einfach durch eine neue Kapsel ausgetauscht. Im Falle des Depots 10 in Form einer Beschichtung ist eine Erneuerung dieser Beschichtung erforderlich. Dies kann entweder vor Ort von einem Zahnarzt oder in einem Labor durchgeführt werden.

In einer bevorzugten Ausführungsform kann das Depot 10 eine optisch ansprechende Form aufweisen, oder es kann die Ausgestaltung eines Schmuckstücks haben, um den ästhetischen Eindruck zu verbessern.

Es wird davon ausgegangen, dass auf der Grundlage der hier beschriebenen spezifischen Struktur viele Variationen vorgenommen werden können, ohne über den Rahmen der Erfindung, wie in den folgenden Ansprüchen definiert, hinauszugehen.

### REFERENZLISTE:

- Depot: 10
- Depots: 10.1, 10.2

- zahnmedizinische Apparatur Zahnimplantat: 20
- inneres Gewinde: 22
- Implantatkörper: 24

- Zahnspange: 30
- Bracket: 32
- Mund: 100
- Zahnfleisch: 102
- Kieferknochen: 104

## Patentansprüche

1. Zahnmedizinische Apparatur zur Montage im Mund (100)
**dadurch gekennzeichnet, dass** sie ein antibakteriell wirkendes Depot (10) umfasst, das kontinuierlich abgebbare Silber-Ionen beinhaltet.

2. Zahnmedizinische Apparatur nach Anspruch 1,
**dadurch gekennzeichnet, dass** es sich um eine Zahnspange (30) handelt.

3. Zahnmedizinische Apparatur nach Anspruch 2,
**dadurch gekennzeichnet, dass** das Depot (10) an der Zahnspange (30) in Form einer Kapsel befestigt ist.

4. Zahnmedizinische Apparatur nach Anspruch 2,
**dadurch gekennzeichnet, dass** auf einem Teil der Zahnspange (30) und/oder auf dem Depot (10) die Silber-Ionen als eine Beschichtung aus kolloidalem Silber angebracht oder aufgebracht sind.

5. Zahnmedizinische Apparatur nach Anspruch 1,
**dadurch gekennzeichnet, dass** es sich um ein Zahnimplantat (20) handelt und dass das Depot (10) an dem Zahnimplantat (20) befestigbar ist, um Infektionen während einer Einheilphase zu verhindern.

6. Zahnmedizinische Apparatur nach Anspruch 5,
**dadurch gekennzeichnet, dass** auf einem Teil des Zahnimplantats (30) und/oder auf dem Depot (10) die Silber-Ionen als eine Beschichtung aus kolloidalem Silber angebracht oder aufgebracht sind.

7. Zahnmedizinische Apparatur nach einem der Ansprüche 1 bis 6,
**dadurch gekennzeichnet, dass** die zahnmedizinische Apparatur ein zweites antibakteriell wirkendes Depot (10.2) umfasst, wobei sich zwischen dem Depot (10) und dem zweiten Depot (10.2) nach der Montage in Mund (100) eine niedrige elektrische Spannungsdifferenz ergibt und dadurch eine besonders effektive antibakterielle Wirkung gewährleistet wird.

8. Zahnmedizinische Apparatur nach einem der Ansprüche 1 bis 7,
**dadurch gekennzeichnet, dass** die Silber-Ionen in einer Matrix aus Metall, Kunststoff und/oder Keramik beinhaltet oder eingebettet sind.

9. Zahnmedizinische Apparatur nach einem der Ansprüche 1 bis 7,
**dadurch gekennzeichnet, dass** die Silber-Ionen in Form eines Silbersalzes angebracht sind.

10. Zahnmedizinische Apparatur nach Anspruch 9,
**dadurch gekennzeichnet, dass** es sich bei dem Silbersalz um Silberchlorid (AgCl) handelt.

11. Zahnmedizinische Apparatur nach einem der Ansprüche 1 bis 10,
**dadurch gekennzeichnet, dass** das Depot (10) unabhängig von der zahnmedizinischen Apparatur austauschbar / erneuerbar ist.
